# FASCICULE DE BREVET EUROPEEN

(11) **EP 2 519 218 B1**
(45) Date de publication et mention de la délivrance du brevet: **27.05.2015**
(21) Numéro de dépôt: 10808919.4
(22) Date de dépôt: 22.12.2010
(51) Int. Cl.: A61K 8/38, A61Q 19/00, C07C 409/34

(54) **DÉRIVÉS DE PEROXYDES DE DIBENZOYLE, LEUR PROCÉDÉ DE PRÉPARATION, LES COMPOSITIONS COSMÉTIQUES OU DERMATOLOGIQUES LES CONTENANT**
DIBENZOYLPEROXIDDERIVATE, HERSTELLUNGSVERFAHREN DAFÜR UND KOSMETISCHE ODER DERMATOLOGISCHE ZUSAMMENSETZUNGEN DAMIT
DIBENZOYL PEROXIDE DERIVATIVES, PREPARATION METHOD THEREOF AND COSMETIC OR DERMATOLOGICAL COMPOSITIONS CONTAINING SAME

(30) Priorité: 29.12.2009 FR 0959630
(43) Date de publication de la demande: 07.11.2012
(73) Titulaire: Galderma Research & Development, 06410 Biot (FR)
(72) Inventeur: RODEVILLE, Nicolas, F-06210 Mandelieu (FR); PASCAL, Jean-Claude, F-06100 Nice (FR); BOUIX-PETER, Claire, F-06220 Vallauris (FR)
(74) Mandataire: Sekhri, Redha
(86) Numéro de dépôt international: PCT/FR2010/052876
(87) Numéro de publication internationale: WO 2011/080469

(56) Documents cités:
- US-A- 4 364 940

## Description

### Domaine technique

La présente invention se rapporte à l'utilisation des composés de formule générale (I) suivante :

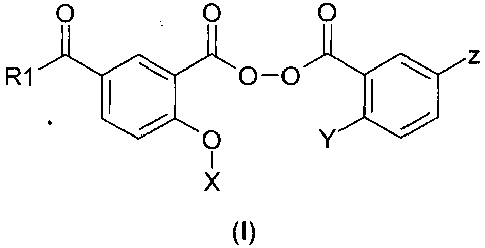

Elle se rapporte également à leur procédé de préparation et à leur application cosmétique ou dermatologique.

Les composés de la présente invention agissent comme agent bactéricide. Ils sont de fait utiles dans le traitement de conditions associées à la présence de bactéries, plus spécifiquement de p acnes.

La présente invention se rapporte également à l'utilisation de composés répondant à la formule générale (I) dans des compositions cosmétiques.

### Etat antérieur de la technique

On sait que la flore cutanée est très variée, aérobie ou anaérobie, composée notamment de staphylocoques epidermidis,aureus et autres microcoques, de corynébactéries aérobies, d'entérobactéries comme Escherichia coli, Klebsiella, Proteus ou de propionobactéries, dans des proportions relatives dépendant de la localisation anatomique, comme mentionné, par exemple, par J. Fleurette dans la Revue du Praticien -30(51) p. 3471-3480 (1980). US 4364940 décrit des composés de peroxydes pour le traitement de l'acné.

Aussi, introduit-on maintenant couramment dans les compositions déodorantes corporelles des agents antimicrobiens qui, en inhibant la prolifération bactérienne. L'utilisation d'agents antimicrobiens en dermatologie et en hygiène corporelle est aussi nécessaire.

La présente invention a ainsi pour objet des composés ayant une action antimicrobienne et destinés à une application cutanée de formule générale (I) suivante : 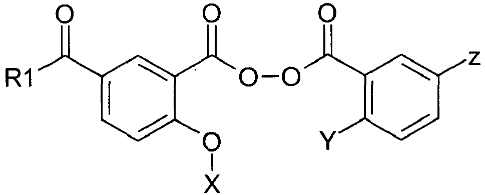 dans laquelle :
- R1 représente un alkyle inférieur ou un alkyle supérieur
- X représente un hydrogène ou l'un des enchaînements suivant : 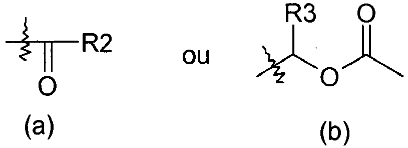 Si X représente (a), R2 représente un alkyle inférieur, un alkyle supérieur, un cycloalkyle, un cycloalkylalkyle, un alkoxy inférieur, un alkoxy supérieur, un cycloalkyloxy, un cycloalkylalkoxy, un aryle ou un aryloxy; Si X représente (b), R3 représente un hydrogène ou un alkyle inférieur
- Y représente un hydrogène ou l'enchaînement suivant : 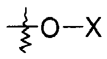
- Z représente un hydrogène ou l'enchaînement suivant :

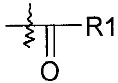

Selon la présente invention, les composés préférés répondant à la formule générale (I) sont ceux qui présentent les caractéristiques suivantes :
- R1 représente une chaîne heptyle linéaire
- X représente un hydrogène ou l'un des enchaînements suivant : 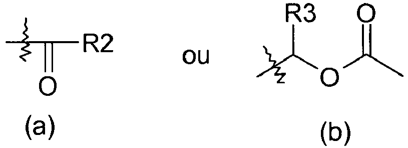 Si X représente (a), R2 représente un alkyle inférieur, un alkyle supérieur, un cycloalkyle, un cycloalkylalkyle, un alkoxy inférieur, un alkoxy supérieur, un cycloalkyloxy, un cycloalkylalkoxy, un aryle ou un aryloxy;
   Si X représente (b), R3 représente un hydrogène ou un groupement méthyle
- Y représente un hydrogène ou l'enchaînement suivant : 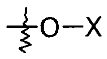
- Z représente un hydrogène ou l'enchaînement suivant :

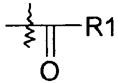

Toujours selon la présente invention, les composés particulièrement préférés répondant à la formule générale (I) sont ceux pour lesquels:
- R1 représente une chaîne heptyle linéaire
- X représente un hydrogène ou l'un des enchaînements suivant : 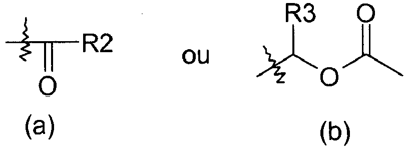 Si X représente (a), R2 représente un alkyle inférieur, un alkyle supérieur, un cycloalkyle, un alkoxy inférieur, ou un alkoxy supérieur ;
   Si X représente (b), R3 représente un hydrogène ou un groupement méthyle
- Y représente un hydrogène ou l'enchaînement suivant : 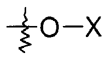
- Z représente un hydrogène

Selon la présente invention, on désigne par un alkyle inférieur, une chaîne hydrocarbonée saturée, linéaire ou ramifiée, comprenant de 1 à 4 atomes de carbone.

Selon la présente invention, on désigne par un alkyle supérieur, une chaîne hydrocarbonée saturée, linéaire ou ramifiée, comprenant de 5 à 10 atomes de carbone.

Selon la présente invention, on désigne par un cycloalkyle, une chaîne hydocarbonée saturée, cyclique, bicyclique ou tricyclique, comprenant 3 à 10 atomes de carbone.

Selon la présente invention, on désigne par un cycloalkylalkyle, par un alkyle substitué par un cycloalkyle.

Selon la présente invention, on désigne par un aryle, un phenyle ou un naphtyle non substitué.

Selon la présente invention, on désigne par un alkoxy inférieur, un atome d'oxygène substitué par un alkyle inférieur.

Selon la présente invention, on désigne par un alkoxy supérieur, un atome d'oxygène substitué par un alkyle supérieur.

Selon la présente invention, on désigne par un cycloalkoxy aryle un atome d'oxygène substitué par un un cycloalkyle.

Selon la présente invention, on désigne par un cycloalkylalkoxy, un atome d'oxygène substitué par un cycloalkylalkyle.

Selon la présente invention, on désigne par un aryloxy un atome d'oxygène substitué par un aryle.

Parmi les composés de formule générale (I) entrant dans le cadre de la présente invention, on peut notamment citer les suivants :
Exemple 1 : peroxyde de (2-acétoxy-5-octanoyl-benzoyl) benzoyle
Exemple 2 : peroxyde de (2-éthoxycarbonyloxy-5-octanoyl-benzoyl) benzoyle
Exemple 3 : Peroxyde de (2-propionyloxy-5-octanoyl-benzoyl) benzoyle
Exemple 4 : Peroxyde de (2-butyryloxy-5-octanoyl-benzoyl) benzoyle
Exemple 5 : Peroxyde de (2-isobutyryloxy-5-octanoyl-benzoyl) benzoyle
Exemple 6 : Peroxyde de [2-(2,2-diméthyl-propionyloxy)-5-octanoyl-benzoyl] benzoyle
Exemple 7 : Peroxyde de (2-pentanoyloxy-5-octanoyl-benzoyl) benzoyle
Exemple 8 : Peroxyde de [2-(2-méthyl-butyryloxy)-5-octanoyl-benzoyl] benzoyle
Exemple 9 : Peroxyde de [2-(3-méthyl-butyryloxy)-5-octanoyl-benzoyl] benzoyle
Exemple 10 : Peroxyde de (2-hexanoyloxy-5-octanoyl-benzoyl) benzoyle
Exemple 11 : Peroxyde de [2-(2-éthyl-butyryloxy)-5-octanoyl-benzoyl] benzoyle
Exemple 12 : Peroxyde de [2-(3,3-diméthyl-butyryloxy)-5-octanoyl-benzoyl] benzoyle
Exemple 13 : Peroxyde de (2-heptanoyloxy-5-octanoyl-benzoyl) benzoyle
Exemple 14 : Peroxyde de (2-octanoyloxy-5-octanoyl-benzoyl) benzoyle
Exemple 15 : Peroxyde de (2-nonanoyloxy-5-octanoyl-benzoyl) benzoyle
Exemple 16 : Peroxyde de (2-cyclopropanecarbonyloxy-5-octanoyl-benzoyl) benzoyle
Exemple 17 : Peroxyde de (2-cyclobutanecarbonyloxy-5-octanoyl-benzoyl) benzoyle
Exemple 18 : Peroxyde de (2-cyclopentanecarbonyloxy-5-octanoyl-benzoyl) benzoyle
Exemple 19 : Peroxyde de (2-cyclohexanecarbonyloxy-5-octanoyl-benzoyl) benzoyle
Exemple 20 : Peroxyde de (2-benzoyloxy-5-octanoyl-benzoyl) benzoyle
Exemple 21 : Peroxyde de [2-(adamantane-1-carbonyloxy)-5-octanoyl-benzoyl] benzoyle
Exemple 22 : Peroxyde de [2-(2-adamantan-1-yl-acétoxy)-5-octanoyl-benzoyl] benzoyle
Exemple 23 : Peroxyde de (2-méthoxycarbonyloxy-5-octanoyl-benzoyl) benzoyle
Exemple 24 : Peroxyde de (2-propoxycarbonyloxy-5-octanoyl-benzoyl) benzoyle
Exemple 25 Peroxyde de (2-isopropoxycarbonyloxy-5-octanoyl-benzoyl) benzoyle
Exemple 26 : Peroxyde de (2-tert-butoxycarbonyloxy-5-octanoyl-benzoyl) benzoyle
Exemple 27 : Peroxyde de (2-butoxycarbonyloxy-5-octanoyl-benzoyl) benzoyle
Exemple 28 : Peroxyde de (2-sec-butoxycarbonyloxy-5-octanoyl-benzoyl) benzoyle
Exemple 29 : Peroxyde de (2-isobutoxycarbonyloxy-5-octanoyl-benzoyl) benzoyle
Exemple 30 : Peroxyde de (2-pentoxycarbonyloxy-5-octanoyl-benzoyl) benzoyle
Exemple 31 : Peroxyde de [2-(1-éthyl-propoxycarbonyloxy)-5-octanoyl-benzoyl] benzoyie
Exemple 32 : Peroxyde de [2-(2,2-diméthyl-propoxycarbonyloxy)-5-octanoyl-benzoyl] benzoyle
Exemple 33 : Peroxyde de (2-hexyloxycarbonyloxy-5-octanoyl-benzoyl) benzoyle
Exemple 34 : Peroxyde de (2-heptyloxycarbonyloxy-5-octanoyl-benzoyl) benzoyle
Exemple 35 : Peroxyde de (2-octyloxycarbonyloxy-5-octanoyl-benzoyl) benzoyle
Exemple 36 : Peroxyde de (2-cyclopropoxycarbonyloxy-5-octanoyl-benzoyl) benzoyle
Exemple 37 : Peroxyde de (2-cyclobutoxycarbonyloxy-5-octanoyl-benzoyl) benzoyle
Exemple 38 : Peroxyde de (2-cyclopentoxycarbonyloxy-5-octanoyl-benzoyl) benzoyle
Exemple 39 : Peroxyde de (2-cyclohexyloxycarbonyloxy-5-octanoyl-benzoyl) benzoyle
Exemple 40 : Peroxyde de (2-phénoxycarbonyloxy-5-octanoyl-benzoyl) benzoyle
Exemple 41 : Peroxyde de (2-acétoxymethoxy-5-octanoyl-benzoyl) benzoyle
Exemple 42 : Peroxyde de [2-(1-acétoxy-éthoxy)-5-octanoyl-benzoyl] benzoyle
Exemple 43 : Peroxyde de bis(2-acétoxy-5-octanoyl)-benzoyle
Exemple 44 : Peroxyde de bis(2-propionyloxy-5-octanoyl)-benzoyle
Exemple 45 : Peroxyde de bis(2-butyryloxy-5-octanoyl)-benzoyle
Exemple 46 : Peroxyde de bis(2-isobutyryloxy-5-octanoyl)-benzoyle
Exemple 47 : Peroxyde de bis[2-(2,2-diméthyl-propionyloxy)-5-octanoyl]-benzoyle
Exemple 48 : Peroxyde de bis(2-pentanoyloxy-5-octanoyl)-benzoyle
Exemple 49 : Peroxyde de bis[2-(2-méthyl-butyryloxy)-5-octanoyl]-benzoyle
Exemple 50 : Peroxyde de bis[2-(3-méthyl-butyryloxy)-5-octanoyl]-benzoyle
Exemple 51 : Peroxyde de bis(2-hexanoyloxy-5-octanoyl)-benzoyle
Exemple 52 : Peroxyde de bis[2-(2-éthyl-butyryloxy)-5-octanoyl]-benzoyle
Exemple 53 : Peroxyde de bis[2-(3,3-diméthyl-butyryloxy)-5-octanoyl]-benzoyle
Exemple 54 : Peroxyde de bis(2-heptanoyloxy-5-octanoyl)-benzoyle
Exemple 55 : Peroxyde de bis(2-octanoyloxy-5-octanoyl)-benzoyle
Exemple 56 : Peroxyde de bis(2-nonanoyloxy-5-octanoyl)-benzoyle
Exemple 57 : Peroxyde de bis(2-cyclopropanecarbonyloxy-5-octanoyl)-benzoyle
Exemple 58 : Peroxyde de bis(2-cyclobutanecarbonyloxy-5-octanoyl)-benzoyle
Exemple 59 : Peroxyde de bis(2-cyclopentanecarbonyloxy-5-octanoyl)-benzoyle
Exemple 60 : Peroxyde de bis(2-cyclohexanecarbonyloxy-5-octanoyl)-benzoyle
Exemple 61 : Peroxyde de bis(2-benzoyloxy-5-octanoyl)-benzoyle
Exemple 62 : Peroxyde de bis[2-(adamantane-1-carbonyloxy)-5-octanoyl]-benzoyle
Exemple 63 : Peroxyde de bis[2-(2-adamantan-1-yl-acétoxy)-5-octanoyl]-benzoyle
Exemple 64 : Peroxyde de bis(2-méthoxycarbonyloxy-5-octanoyl)-benzoyle
Exemple 65 : Peroxyde de bis(2-éthoxycarbonyloxy-5-octanoyl)-benzoyle
Exemple 66 : Peroxyde de bis(2-propoxycarbonyloxy-5-octanoyl)-benzoyle
Exemple 67 : Peroxyde de bis(2-isopropoxycarbonyloxy-5-octanoyl)-benzoyle
Exemple 68 : Peroxyde de bis(2-tert-butoxycarbonyloxy-5-octanoyl)-benzoyle
Exemple 69 : Peroxyde de bis(2-butoxycarbo.nyloxy-5-octanoyl)-benzoyle
Exemple 70 : Peroxyde de bis(2-sec-butoxycarbonyloxy-5-octanoyl)-benzoyle
Exemple 71 : Peroxyde de bis(2-isobutoxycarbonyloxy-5-octanoyl)-benzoyle
Exemple 72 : Peroxyde de bis(2-pentoxycarbonyloxy-5-octanoyl)-benzoyle
Exemple 73 : Peroxyde de bis[2-(1-éthyl-propoxycarbonyloxy)-5-octanoyl]-benzoyle
Exemple 74 : Peroxyde de bis[2-(2,2-diméthyl-propoxycarbonyloxy)-5-octanoyl]-benzoyle
Exemple 75 : Peroxyde de bis(2-hexyloxycarbonyloxy-5-octanoyl)-benzoyle
Exemple 76 : Peroxyde de bis(2-heptyloxycarbonyloxy-5-octanoyl)-benzoyle
Exemple 77 : Peroxyde de bis(2-octyloxycarbonyloxy-5-octanoyl)-benzoyle
Exemple 78 : Peroxyde de bis(2-cyclopropoxycarbonyloxy-5-octanoyl)-benzoyle
Exemple 79 : Peroxyde de bis(2-cyclobutoxycarbonyloxy-5-octanoyl)-benzoyle
Exemple 80 : Peroxyde de bis(2-cyclopentoxycarbonyloxy-5-octanoyl)-benzoyle
Exemple 81 : Peroxyde de bis(2-cyclohexyloxycarbonyloxy-5-octanoyl)-benzoyle
Exemple 82 : Peroxyde de bis(2-phénoxycarbonyloxy-5-octanoyl)-benzoyle
Exemple 83 : Peroxyde de bis(2-acétoxyméthoxy-5-octanoyl)-benzoyle
Exemple 84 : Peroxyde de bis[2-(1-acétoxy-éthoxy)-5-octanoyl]-benzoyle

Une description générale de méthodes de préparation des composés de formule (I) est donnée ci-après. Sur ces schémas et dans la description du procédé qui va suivre, à moins qu'il n'en soit spécifié autrement, tous les substituants sont tels que définis pour les composés de formule (I).

Dans le cas où le groupement Y défini dans la formule (I) est un hydrogène et le groupement Z défini dans la formule (I) est un hydrogène, les composés de formule générale (I) sont préparés suivant le schéma réactionnel 1 ou le schéma réactionnel 2 présentés ci-dessous.

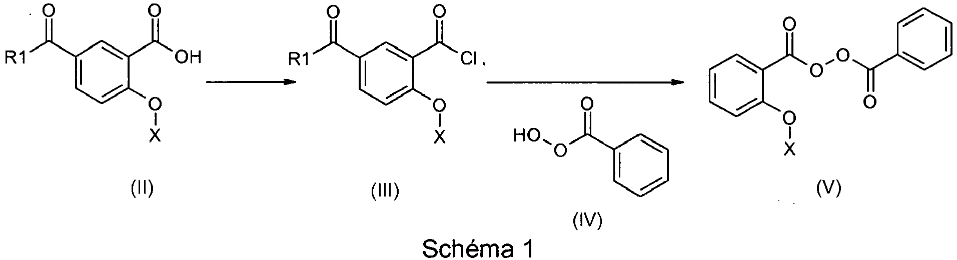

Selon le schéma 1, les chlorures d'acides de formule générale (III) sont préparés à partir de l'acide carboxylique (II), par des méthodes choisies parmi celles connues de l'homme de l'art. Elles comprennent l'utilisation de chlorure de thionyle et de pyridine dans un solvant tel que le toluène ou le dichlorométhane par exemple.

Les acides carboxyliques de formule générale (II) sont disponibles commercialement ou sont préparés selon les méthodes décrites dans le schéma 5 .

Dans une dernière étape, les composés de formule générale (V) peuvent être préparés par couplage entre les chlorures d'acyles de formule (III) et le peracide de formule (IV), en utilisant comme base de la pyridine dans un mélange de solvants tel que le dichlorométhane et le chlororforme.

Le peracide de formule générale (IV) est préparé selon la méthode décrite dans le schéma 6 à partir du peroxyde de benzoyle.

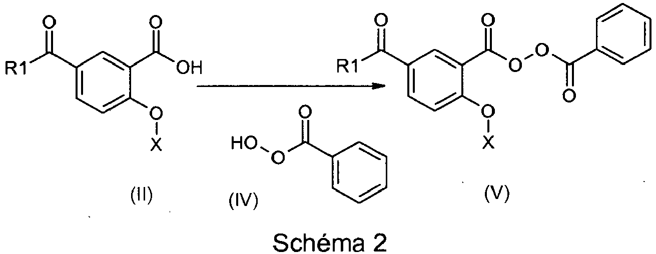

Selon le schéma 2, les peroxydes de formule générale (V) sont préparés par couplage entre les acides carboxyliques de formule (II) et le peracide de formule (IV), en utilisant par exemple comme agent de couplage le N,N'-dicyclohexylcarbodiimide par exemple dans un mélange de solvants tel que éther diéthylique et dichlorométhane.

Les acides carboxyliques de formule générale (II) sont disponibles commercialement ou sont préparés selon les méthodes décrites dans le schéma 5.

Le peracide de formule générale (IV) est préparé selon la méthode décrite dans le schéma 6 à partir du peroxyde de benzoyle.

Dans le cas où les groupements Y et Z définis dans la formule (I) ne sont pas un hydrogène, les composés de formule générale (I) sont préparés suivants le schéma réactionnel 3 ou le schéma réactionnel 4 présentés ci-dessous.

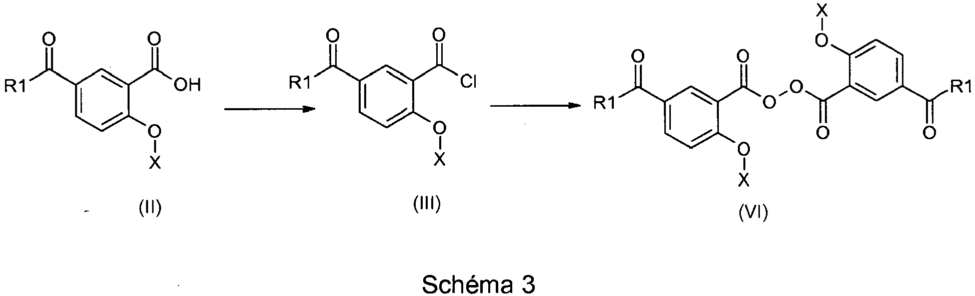

Selon le schéma 3, les chlorures d'acides de formule générale (III) sont préparés à partir de l'acide carboxylique (II), par des méthodes choisies parmi celles connues de l'homme de l'art. Elles comprennent l'utilisation de chlorure de thionyle et de pyridine dans un solvant tel que le toluène ou le dichlorométhane par exemple.

Les acides carboxyliques de formule générale (II) sont préparés selon les méthodes décrites dans le schéma 5.

Dans une dernière étape, les composés de formule générale (VI) peuvent être préparés par couplage entre deux chlorures d'acyles de formule (III) par des méthodes choisies parmi celles connues de l'homme de l'art. Elles comprennent l'utilisation de peroxyde d'hydrogène et de bicarbonate de sodium dans un solvant tel que le tétrahydrofuranne par exemple.

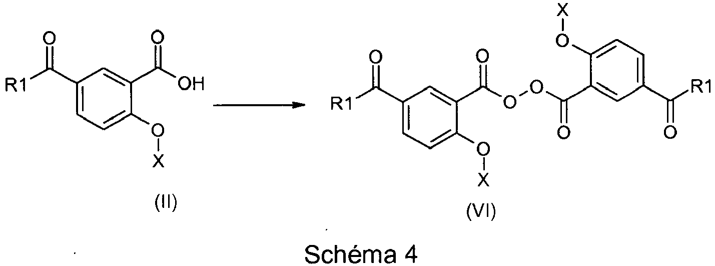

Selon le schéma 4, les peroxydes de formule générale (VI) sont préparés par réaction entre deux acides carboxyliques de formule (II), en utilisant par exemple du N,N'-dicyclohexylcarbodiimide et du peroxyde d'hydrogène dans un mélange de solvants tel que éther diéthylique et dichlorométhane.

Dans le cas où X n'est pas un hydrogène, les acides carboxyliques de formule (II) peuvent être préparés selon le schéma réactionnel 5.

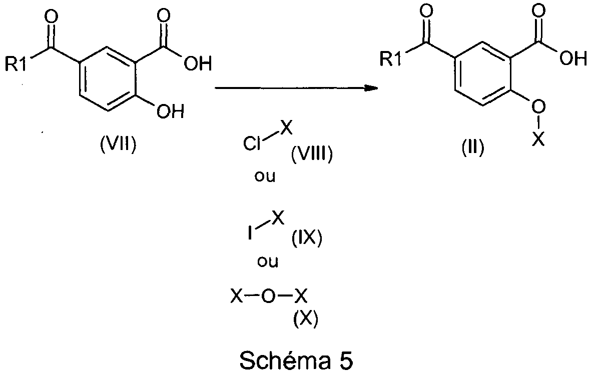

Selon le schéma 5, les acides carboxyliques de formule (II) sont préparés à partir de l'acide carboxylique (VII) par des méthodes choisies parmi celles connues de l'homme de l'art. Elles comprennent l'utilisation d'halogénures de formule (VIII) et (IX) ou d'anhydrides de formule (X), et de bases telles que la N,N-diméthylaniline, la triéthylamine, la pyridine, le carbonate de potassium, dans un solvant tel que le toluène ou le dichlorométhane par exemple.

Les halogénures de formule (VIII) et (IX) ainsi que les anhydrides de formule (X) sont disponibles commercialement.

Dans le cas où X est un hydrogène les acides carboxyliques de formule (II) sont disponibles commercialement.

Le peracide de formule (IV) peut être préparé selon le schéma réactionnel 6.

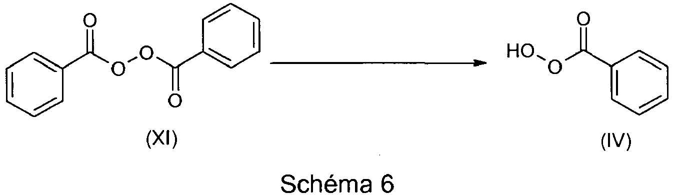

Selon le schéma 6, le peracide de formule (IV) est préparé à partir du peroxyde de dibenzoyle (XI) par des méthodes choisies parmi celles connues de l'homme de l'art. Elles comprennent l'utilisation d'un peroxyde (XI) et de sodium dans un mélange de solvants tel que le méthanol et le chloroforme par exemple.

### Etude de la sensibilité des peroxydes versus le peroxyde de dibenzoyle sur Propionibacterium acnes.

### Principe du test :

Le but est d'évaluer l'activité anti-bactérienne des peroxydes par la mesure des Concentrations Minimales Inhibitrices (CMI). La CMI est définie comme la plus faible concentration de produit capable d'inhiber toute croissance visible à l'oeil nu. Les CMI sont déterminées par une méthode de référence internationale : « Methods For Antimicrobial Susceptibility Testing of Anaerobic Bacteria ; Approved Standard, Seven Edition » M11A7 du CLSI (Clinical Laboratory Standards institute), méthode de dilution en milieu gélosé.

### Souche microbienne et origine :

L'étude de la sensibilité des produits est étudiée sur dix souches de *P. acnes* isolées de prélèvements pathologiques humains. Afin de s'assurer de la validité des résultats obtenus et de leur reproductibilité, 3 souches de référence préconisées par la norme M 11 A7 sont introduites dans chaque série d'essais : *Bacteroides fragilis* ATCC 25285, *Bacteroides thetaiotaomicron* ATCC 29741, *Eubacterium lentum* ATCC 43055, *Clostridium difficile* ATCC 700057.

### Test des produits :

Les produits sont solubilisés à 1280 mg/L dans un mélange Ethanol absolu/ Tween 80 / milieu de culture Brucella stérile (5/10/85 v/v/v). Des dilutions successives au demi sont réalisées en eau distillée stérile à partir de cette solution-mère et selon les directives d'Ericsson et Sherris. La gamme est composée de 8 concentrations de 10 mg/L à 1280 mg/L à intervalle de raison 2.

Une préculture en milieu de Rosenow est diluée en bouillon Brucella de façon à obtenir une opacité voisine de l'échelle 0,5 de McFarland. L'inoculum contient alors 10⁷ à 10⁸ UFC/ml.

À l'aide d'un ensemenceur à têtes multiples de type Steers, 2 à 3 µl de l'inoculum, préalablement déposé dans chacune des cupules de l'ensemenceur, sont déposés sur les boites de Pétri. L'inoculum final est d'environ 10⁵ UFC par spot d'inoculation.

La lecture des résultats est réalisée après 48 h d'incubation en chambre anaérobie. La CMI est la plus faible concentration de produit capable d'inhiber toute croissance visible.

| Souche testée | Peroxyde de Benzoyle CMI en mg/L | Exemple n°1 CMI en mg/L |
|---|---|---|
| CSS 2971 | 640 | 320 |
| CSS 2913 | 640 | 320 |
| CSS 3288 | 640 | 320 |
| 1044 | 640 | 320 |
| 1045 | 640 | 320 |
| 1201 | 640 | 320 |
| 3069 | 640 | 1280 |
| 998 | 640 | 1280 |
| UAA 2281 | 640 | 320 |
| UAA 2285 | 640 | 320 |

### Évaluation de l'effet bactéricide des molécules versus le peroxyde de dibenzoyle sur Propionibacterium acnes.

**Principe du test :** le but est d'évaluer l'effet bactéricide à partir de la mesure de la Concentration Minimale Inhibitrice (CMI) préalablement réalisée. La méthodologie proposée de l'évaluation de la bactéricidie est inspirée des normes NF EN 1040 « Quantitative suspension test for the evaluation of basic bactericidal activity of chemical disinfectants and antiseptics » et T72-300 « Essai de suspension par dilution-neutralisation - Détermination de l'efficacité des produits sur divers microorganisme dans les conditions pratiques d'emploi ».

Les antiseptiques agissant après un temps de contact, il est nécessaire de bloquer leur action avec un agent neutralisant.

### Souche microbienne et origine :

L'évaluation de l'effet bactéricide est réalisée sur une souche de *P. acnes* UAA 2284 (isolée de prélèvements pathologiques humains).

### Test des produits :

Au préalable, un tube témoin de milieu de culture Brucella et un tube contenant la solution neutralisante sont préparés. Les produits sont solubilisés dans un mélange Ethanol absolu/ Tween 80 (7/3 v/v). Les dilutions suivantes sont effectuées dans l'eau distillée stérile. La concentration utilisée dans chaque essai est selon les essais d'une fois à quatre fois la CMI.

La souche de P. acnes est inoculée en bouillon Brucella puis incubée à 35-36°C pendant 48 heures. Une dilution de cette préculture est réalisée ensuite en bouillon Brucella de façon à obtenir un trouble équivalent à celui du tube étalon 0.5 de la gamme de McFarland soit un inoculum d'environ 10⁷-10⁸ UFC/ml. Un millilitre de tube témoin (contrôle ou neutralisant) ou un millilitre de la solution de produit à tester 10X (essai) est mis en contact avec 9 ml de l'inoculum préparé précédemment : soit un inoculum final dans le tube test d'environ 10⁷ UFC/ml. Après 0, 15 mn, 30 mn, 45 mn et une heure de contact avec le produit testé 100 microlitres du tube essai sont prélevés et mis immédiatement dans 900µl de solution neutralisante. Un dénombrement des survivants est alors réalisé et la bactéricidie est définie comme étant une réduction de trois logarithmes log₁₀ de l'inoculum de départ (figure 1 ci-dessous).

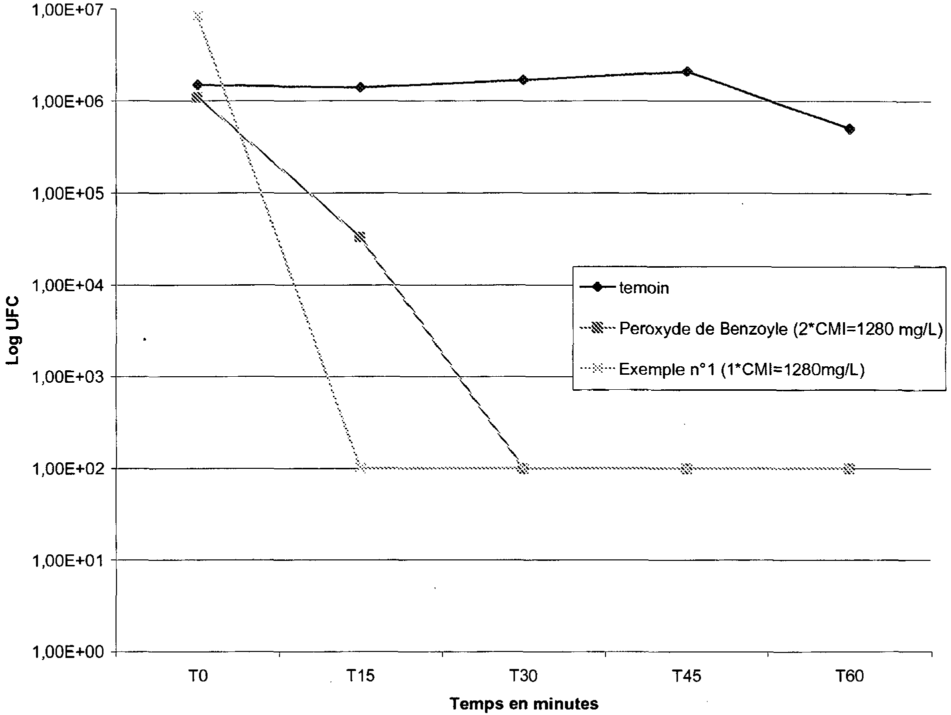

### Exemple 1 : peroxyde de (2-acetoxy-5-octanoyl-benzoyl) benzoyle

### 1-1 : acide 2-acétoxy-5-octanoyl-benzoique

6g (22.7 mmoles) d'acide 2-hydroxy-5-octanoyl-benzoïque et 12 mL(0.145 moles) de pyridine sont dissous dans 15 mL d'acétone. Le milieu est refroidi à 0°C puis 12 mL (0.166 moles) de chlorure d'acétyle sont additionnés goutte à goutte. Après 15 heures d'agitation à température ambiante, de l'eau est rajoutée et le mélange est extrait par du dichlorométhane. La phase organique est séchée sur sulfate de magnésium, filtrée puis concentrée. 7g d'acide 2-acétoxy-5-octanoyl-benzoïque sont obtenus sous forme de solide blanc avec un rendement quantitatif.

### 1-2 : Chlorure de 2-acétoxy-5-octanoyl-benzoyle

5.5g (18 mmoles) d'acide 2-acétoxy-5-octanoyl-benzoïque sont dissous dans 55ml de dichlorométhane avec une goutte de pyridine. 1.6ml (21.5 mmoles) de chlorure de thionyle sont additionnés goutte à goutte et le mélange est agité à 35°C pendant 18h puis concentré à sec. 5,6g de chlorure de 2-acétoxy-5-octanoyl-benzoyle sont obtenus sous forme d'une huile rouge avec un rendement de 96%.

### 1-3 : Acide perbenzoïque

19g (78 mmoles) de dibenzylperoxyde sont solubilisés dans 125ml de chloroforme à -5°C. 2,2g (94 mmoles) de sodium dissous dans 50ml de méthanol sous flux d'azote, sont additionnés goutte à goutte. Après 30 minutes d'agitation à -5°C, de l'eau glacée est rajoutée et le milieu est acidifiée avec une solution aqueuse d'acide sulfurique 2N. Une extraction au dichlorométhane est effectuée puis la phase organique est séchée sur sulfate de magnésium, filtrée et concentrée. 9g d'acide perbenzoïque sont obtenus sous forme de solide blanc avec un rendement de 83%.

### 1-4 : peroxyde de (2-acetoxy-5-octanoyl-benzoyl) benzoyle

5.5g (16.9 mmoles) de chlorure de 2-acétoxy-5-octanoyl-benzoyle et 3.5g (25.4 mmoles) d'acide perbenzoïque sont dissous dans 22ml de chloroforme. Le mélange est refroidi à -18°C puis 1.14g (14.4 mmoles) de pyridine dans 3ml de dichlorométhane sont ajoutés goutte à goutte. Après 1 heure d'agitation à -18°C, de l'eau est rajoutée et le mélange est extrait par du dichlorométhane. La phase organique est séchée sur sulfate de sodium, filtrée puis concentrée. Le résidu est purifié par chromatographie sur gel de silice élué avec un mélange pentane / dichlorométhane, 1/1. Le solide jaune obtenu est précipité dans du pentane à -18°C. Le précipité est filtré puis rincé avec du pentane et séché. 2g de peroxyde de (2-acetoxy-5-octanoyl)-benzoyle sont obtenus sous forme de poudre blanche avec un rendement de 28%.

RMN ¹H 300 MHz /CDCl₃ : δ = 0,91 (m, 3H) ; 1,37 (m, 8H) ; 1,77 (m, 2H) ; 2,41 (s, 3H), 3,02 (t, J=10 Hz, 2H) ; 7,35 (d, J=12 Hz, 1H); 7,55 (dd, J=12 Hz, 2H) ; 7,72 (m, 1 H) ; 8.09 (d, J=12 Hz, 2H) ; 8,27 (d, J=12 Hz, 1 H) ; 8,63 (s, 1 H)

### Exemple 2 : peroxyde de (2-éthoxycarbonyloxy-5-octanoyl-benzoyl) benzoyle

### 2-1 : Acide de 2-éthoxycarbonyloxy-5-octanoyl-benzoïque

4.89g (18.50 mmoles) d'acide 2-hydroxy-5-octanoyl-benzoïque et 9ml (70.30 mmoles) de N,N-diméthylaniline sont dissous dans 30ml de toluène. Le milieu est refroidi à 0°C puis 1.77ml (18.50 mmoles) de chloroformate d'éthyle sont additionnés goutte à goutte. Après 2 heures d'agitation à température ambiante, le mélange est lavé avec une solution aqueuse d'acide chlorhydrique 0.5N puis avec de l'eau. La phase organique est séchée sur sulfate de magnésium, filtrée et concentrée. 5.59 g d'acide de 2-éthoxycarbonyloxy-5-octanoyl-benzoïque sont obtenus sous forme de solide blanc avec un rendement de 89%.

### 2-2 : Chlorure de 2- éthoxycarbonyloxy -5-octanoyl-benzoyle

5,59g (16.6 mmoles) d'acide de 2-éthoxycarbonyloxy-5-octanoyl-benzoïque sont dissous dans 56ml de toluène avec quelques gouttes de pyridine. 1.45 ml (20 mmoles) de chlorure de thionyle sont additionnés goutte à goutte et le mélange est agité à 55°C pendant 14h puis concentré à sec. 5.79 g de chlorure de 2-éthoxycarbonyloxy -5-octanoyl-benzoyle avec un rendement de 98%.

### 2-3 : peroxyde de (2-éthoxycarbonyloxy-5-octanoyl-benzoyl) benzoyle

De manière analogue à l'exemple 1-4, à partir de 5.79g (16.3 mmoles) de chlorure de 2-éthoxycarbonyloxy-5-octanoyl-benzoyle et 3.38g (24.5 mmoles) d'acide perbenzoïque (préparé comme décrit dans l'exemple 1-3), 2g de peroxyde de (2-éthoxycarbonyloxy-5-octanoyl)-benzoyle sont obtenus sous forme d'une huile incolore avec un rendement de 26%.

RMN ¹H 300 MHz/CDCl₃ : δ = 0,88 (m, 3H) ; 1,20 (m, 11 H) ; 1,78 (m, 2H) ; 3,00 (t, J=6Hz, 2H) ; 4.43 (q, J=7 Hz, 2H) ; 7,42 (d, J=12 Hz, 1H); 7,55 (t, J=12 Hz, 2H) ; 7,72 (m, 1 H) ; 8.10 (d, J=12 Hz, 2H) ; 8,27 (d, J=12 Hz, 1 H) ; 8,60 (s, 1 H).

## Revendications

1. Composés de formule générale (I) suivante : 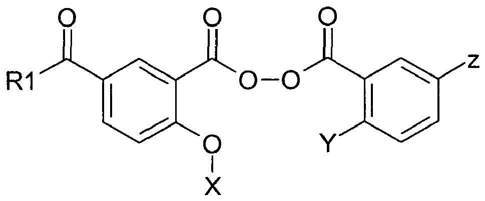 dans laquelle :
- R1 représente un alkyle inférieur ou un alkyle supérieur
- X représente un hydrogène ou l'un des enchaînements suivant : 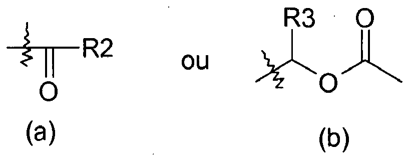
Si X représente (a), R2 représente un alkyle inférieur, un alkyle supérieur, un cycloalkyle, un cycloalkylalkyle, un alkoxy inférieur, un alkoxy supérieur, un cycloalkyloxy, un cycloalkylalkoxy, un aryle ou un aryloxy;
Si X représente (b), R3 représente un hydrogène ou un alkyle inférieur
- Y représente un hydrogène ou l'enchaînement suivant : 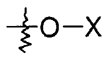
- Z représente un hydrogène ou l'enchaînement suivant :

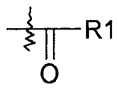

2. Composés selon la revendication 1 **caractérisés en ce que**
- R1 représente une chaîne heptyle linéaire
- X représente un hydrogène ou l'un des enchaînements suivant : 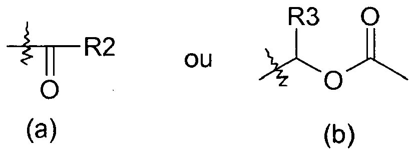 Si X représente (a), R2 représente un alkyle inférieur, un alkyle supérieur, un cycloalkyle, un cycloalkylalkyle, un alkoxy inférieur, un alkoxy supérieur, un cycloalkyloxy, un cycloalkylalkoxy, un aryle ou un aryloxy;
Si X représente (b), R3 représente un hydrogène ou un groupement méthyle
- Y représente un hydrogène ou l'enchaînement suivant : 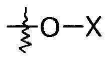
- Z représente un hydrogène ou l'enchaînement suivant :

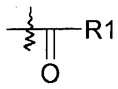

3. Composés selon les revendications 1 ou 2**caractérisés en ce que**
R1 représente une chaîne heptyle linéaire
- X représente un hydrogène ou l'un des enchaînements suivant : 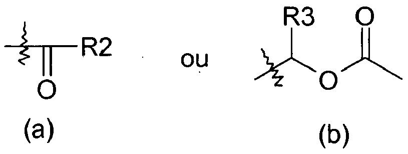
Si X représente (a), R2 représente un alkyle inférieur, un alkyle supérieur, un cycloalkyle, un alkoxy inférieur, ou un alkoxy supérieur ;
Si X représente (b), R3 représente un hydrogène ou un groupement méthyle
- Y représente un hydrogène ou l'enchaînement suivant : 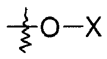
- Z représente un hydrogène

4. Composé selon l'une des revendications 1 à 3 choisi dans la liste constituée par les composés suivants :
Exemple 1 : peroxyde de (2-acétoxy-5-octanoyl-benzoyl) benzoyle
Exemple 2 : peroxyde de (2-éthoxycarbonyloxy-5-octanoyl-benzoyl) benzoyle
Exemple 3 : Peroxyde de (2-propionyloxy-5-octanoyl-benzoyl) benzoyle
Exemple 4 : Peroxyde de (2-butyryloxy-5-octanoyl-benzoyl) benzoyle
Exemple 5 : Peroxyde de (2-isobutyryloxy-5-octanoyl-benzoyl) benzoyle
Exemple 6 : Peroxyde de [2-(2,2-diméthyl-propionyloxy)-5-octanoyl-benzoyl] benzoyle
Exemple 7 : Peroxyde de (2-pentanoyloxy-5-octanoyl-benzoyl) benzoyle
Exemple 8 : Peroxyde de [2-(2-méthyl-butyryloxy)-5-octanoyl-benzoyl] benzoyle
Exemple 9 : Peroxyde de [2-(3-méthyl-butyryloxy)-5-octanoyl-benzoyl] benzoyle
Exemple 10 : Peroxyde de (2-hexanoyloxy-5-octanoyl-benzoyl) benzoyle
Exemple 11 : Peroxyde de [2-(2-éthyl-butyryloxy)-5-octanoyl-benzoyl] benzoyle
Exemple 12 : Peroxyde de [2-(3,3-diméthyl-butyryloxy)-5-octanoyl-benzoyl] benzoyle
Exemple 13 : Peroxyde de (2-heptanoyloxy-5-octanoyl-benzoyl) benzoyle
Exemple 14 : Peroxyde de (2-octanoyloxy-5-octanoyl-benzoyl) benzoyle
Exemple 15 : Peroxyde de (2-nonanoyloxy-5-octanoyl-benzoyl) benzoyle
Exemple 16 : Peroxyde de (2-cyclopropanecarbonyloxy-5-octanoyl-benzoyl) benzoyle
Exemple 17 : Peroxyde de (2-cyclobutanecarbonyloxy-5-octanoyl-benzoyl) benzoyle
Exemple 18 : Peroxyde de (2-cyclopentanecarbonyloxy-5-octanoyl-benzoyl) benzoyle
Exemple 19 : Peroxyde de (2-cyclohexanecarbonyloxy-5-octanoyl-benzoyl) benzoyle
Exemple 20 : Peroxyde de (2-benzoyloxy-5-octanoyl-benzoyl) benzoyle
Exemple 21 : Peroxyde de [2-(adamantane-1-carbonyloxy)-5-octanoyl-benzoyl] benzoyle
Exemple 22 : Peroxyde de [2-(2-adamantan-1-yl-acétoxy)-5-octanoyl-benzoyl] benzoyle
Exemple 23 : Peroxyde de (2-méthoxycarbonyloxy-5-octanoyl-benzoyl) benzoyle
Exemple 24 : Peroxyde de (2-propoxycarbonyloxy-5-octanoyl-benzoyl) benzoyle
Exemple 25 Peroxyde de (2-isopropoxycarbonyloxy-5-octanoyl-benzoyl) benzoyle
Exemple 26 : Peroxyde de (2-tert-butoxycarbonyloxy-5-octanoyl-benzoyl) benzoyle
Exemple 27 : Peroxyde de (2-butoxycarbonyloxy-5-octanoyl-benzoyl) benzoyle
Exemple 28 : Peroxyde de (2-sec-butoxycarbonyloxy-5-octanoyl-benzoyl) benzoyle
Exemple 29 : Peroxyde de (2-isobutoxycarbonyloxy-5-octanoyl-benzoyl) benzoyle
Exemple 30 : Peroxyde de (2-pentoxycarbonyloxy-5-octanoyl-benzoyl) benzoyle
Exemple 31 : Peroxyde de [2-(1-éthyl-propoxycarbonyloxy)-5-octanoyl-benzoyl] benzoyle
Exemple 32 : Peroxyde de [2-(2,2-diméthyl-propoxycarbonyloxy)-5-octanoyl-benzoyl] benzoyle
Exemple 33 : Peroxyde de (2-hexyloxycarbonyloxy-5-octanoyl-benzoyl) benzoyle
Exemple 34 : Peroxyde de (2-heptyloxycarbonyloxy-5-octanoyl-bénzoyl) benzoyle
Exemple 35 : Peroxyde de (2-octyloxycarbonyloxy-5-octanoyl-benzoyl) benzoyle
Exemple 36 : Peroxyde de (2-cyclopropoxycarbonyloxy-5-octanoyl-benzoyl) benzoyle
Exemple 37 : Peroxyde de (2-cyclobutoxycarbonyloxy-5-octanoyl-benzoyl) benzoyle
Exemple 38 : Peroxyde de (2-cyclopentoxycarbonyloxy-5-octanoyl-benzoyl) benzoyle
Exemple 39 : Peroxyde de (2-cyclohexyloxycarbonyloxy-5-octanoyl-benzoyl) benzoyle
Exemple 40 : Peroxyde de (2-phénoxycarbonyloxy-5-octanoyl-benzoyl) benzoyle
Exemple 41 : Peroxyde de (2-acétoxymethoxy-5-octanoyl-benzoyl) benzoyle
Exemple 42 : Peroxyde de [2-(1-acétoxy-éthoxy)-5-octanoyl-benzoyl] benzoyle
Exemple 43 : Peroxyde de bis(2-acétoxy-5-octanoyl)-benzoyle
Exemple 44 : Peroxyde de bis(2-propionyloxy-5-octanoyl)-benzoyle
Exemple 45 : Peroxyde de bis(2-butyryloxy-5-octanoyl)-benzoyle
Exemple 46 : Peroxyde de bis(2-isobutyryloxy-5-octanoyl)-benzoyle
Exemple 47 : Peroxyde de bis[2-(2,2-diméthyl-propionyloxy)-5-octanoyl]-benzoyle
Exemple 48 : Peroxyde de bis(2-pentanoyloxy-5-octanoyl)-benzoyle
Exemple 49 : Peroxyde de bis[2-(2-méthyl-butyryloxy)-5-octanoyl]-benzoyle
Exemple 50 : Peroxyde de bis[2-(3-méthyl-butyryloxy)-5-octanoyl]-benzoyle
Exemple 51 : Peroxyde de bis(2-hexanoyloxy-5-octanoyl)-benzoyle
Exemple 52 : Peroxyde de bis[2-(2-éthyl-butyryloxy)-5-octanoyl]-benzoyle
Exemple 53 : Peroxyde de bis[2-(3,3-diméthyl-butyryloxy)-5-octanoyl]-benzoyle
Exemple 54 : Peroxyde de bis(2-heptanoyloxy-5-octanoyl)-benzoyle
Exemple 55 : Peroxyde de bis(2-octanoyloxy-5-octanoyl)-benzoyle
Exemple 56 : Peroxyde de bis(2-nonanoyloxy-5-octanoyl)-benzoyle
Exemple 57 : Peroxyde de bis(2-cyclopropanecarbonyloxy-5-octanoyl)-benzoyle
Exemple 58 : Peroxyde de bis(2-cyclobutanecarbonyloxy-5-octanoyl)-benzoyle
Exemple 59 : Peroxyde de bis(2-cyclopentanecarbonyloxy-5-octanoyl)-benzoyle
Exemple 60 : Peroxyde de bis(2-cyclohexanecarbonyloxy-5-octanoyl)-benzoyle
Exemple 61 : Peroxyde de bis(2-benzoyloxy-5-octanoyl)-benzoyle
Exemple 62 : Peroxyde de bis[2-(adamantane-1-carbonyloxy)-5-octanoyl]-benzoyle
Exemple 63 : Peroxyde de bis[2-(2-adamantan-1-yl-acétoxy)-5-octanoyl]-benzoyle
Exemple 64 : Peroxyde de bis(2-méthoxycarbonyloxy-5-octanoyl)-benzoyle
Exemple 65 : Peroxyde de bis(2-éthoxycarbonyloxy-5-octanoyl)-benzoyle
Exemple 66 : Peroxyde de bis(2-propoxycarbonyloxy-5-octanoyl)-benzoyle
Exemple 67 : Peroxyde de bis(2-isopropoxycarbonyloxy-5-octanoyl)-benzoyle
Exemple 68 : Peroxyde de bis(2-tert-butoxycarbonyloxy-5-octanoyl)-benzoyle
Exemple 69 : Peroxyde de bis(2-butoxycarbonyloxy-5-octanoyl)-benzoyle
Exemple 70 : Peroxyde de bis(2-sec-butoxycarbonyloxy-5-octanoyl)-benzoyle
Exemple 71 : Peroxyde de bis(2-isobutoxycarbonyloxy-5-octanoyl)-benzoyle
Exemple 72 : Peroxyde de bis(2-pentoxycarbonyloxy-5-octanoyl)-benzoyle
Exemple 73 : Peroxyde de bis[2-(1-éthyl-propoxycarbonyloxy)-5-octanoyl]-benzoyle
Exemple 74 : Peroxyde de bis[2-(2,2-diméthyl-propoxycarbonyloxy)-5-octanoyl]-benzoyle
Exemple 75 : Peroxyde de bis(2-hexyloxycarbonyloxy-5-octanoyl)-benzoyle
Exemple 76 : Peroxyde de bis(2-heptyloxycarbonyloxy-5-octanoyl)-benzoyle
Exemple 77 : Peroxyde de bis(2-octyloxycarbonyloxy-5-octanoyl)-benzoyle
Exemple 78 : Peroxyde de bis(2-cyclopropoxycarbonyloxy-5-octanoyl)-benzoyle
Exemple 79 : Peroxyde de bis(2-cyclobutoxycarbonyloxy-5-octanoyl)-benzoyle
Exemple 80 : Peroxyde de bis(2-cyclopentoxycarbonyloxy-5-octanoyl)-benzoyle
Exemple 81 : Peroxyde de bis(2-cyclohexyloxycarbonyloxy-5-octanoyl)-benzoyle
Exemple 82 : Peroxyde de bis(2-phénoxycarbonyloxy-5-octanoyl)-benzoyle
Exemple 83 : Peroxyde de bis(2-acétoxyméthoxy-5-octanoyl)-benzoyle
Exemple 84 : Peroxyde de bis[2-(1-acétoxy-éthoxy)-5-octanoyl]-benzoyle

5. Composé selon l'une des revendications 1 à 4 en tant que médicament.

6. Composé selon l'une des revendications 1 à 5 pour le traitement de pathologies ou de désordres liés à la présence de Propionibacterium acnes

7. Composé selon l'une des revendications 1 à 4 pour incorporation à une composition cosmétique, **caractérisée en ce qu'**il inhibe la prolifération des germes pathogènes impliqués dans l'apparition des désordres cutanés acnéiques, notamment **P. acnes**

## Patentansprüche

1. Verbindungen der folgenden allgemeinen Formel (I): 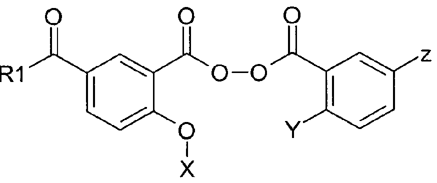 in der:
- R1 für ein niederes Alkyl oder ein höheres Alkyl steht
- X für Wasserstoff oder eine der folgenden Ketten steht: 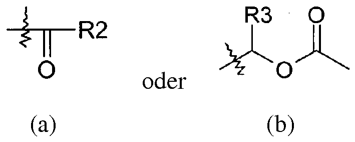
wenn X für (a) steht, steht R2 für ein niederes Alkyl, ein höheres Alkyl, ein Cycloalkyl, ein Cycloalkylalkyl, ein niederes Alkoxy, ein höheres Alkoxy, ein Cycloalkyloxy, ein Cycloalkylalkoxy, ein Aryl oder ein Aryloxy;
wenn X für (b) steht, steht R3 für Wasserstoff oder ein niederes Alkyl
- Y für Wasserstoff oder die folgende Kette steht: 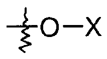
- Z für Wasserstoff oder die folgende Kette steht:

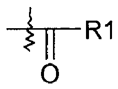

2. Verbindungen gemäß Anspruch 1, **dadurch gekennzeichnet, dass**
- R1 für eine lineare Heptylkette steht
- X für Wasserstoff oder eine der folgenden Ketten steht: 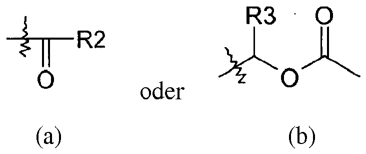 wenn X für (a) steht, steht R2 für ein niederes Alkyl, ein höheres Alkyl, ein Cycloalkyl, ein Cycloalkylalkyl, ein niederes Alkoxy, ein höheres Alkoxy, ein Cycloalkyloxy, ein Cycloalkylalkoxy, ein Aryl oder ein Aryloxy;
wenn X für (b) steht, steht R3 für Wasserstoff oder eine Methylgruppe
- Y für Wasserstoff oder die folgende Kette steht: 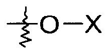
- Z für Wasserstoff oder die folgende Kette steht:

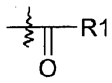

3. Verbindungen gemäß Anspruch 1 oder 2, **dadurch gekennzeichnet, dass**
- R1 für eine lineare Heptylkette steht
- X für Wasserstoff oder eine der folgenden Ketten steht: 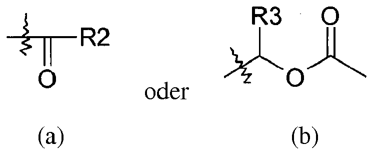 wenn X für (a) steht, steht R2 für ein niederes Alkyl, ein höheres Alkyl, ein Cycloalkyl, ein niederes Alkoxy oder ein höheres Alkoxy;
wenn X für (b) steht, steht R3 für Wasserstoff oder eine Methylgruppe
- Y für Wasserstoff oder die folgende Kette steht: 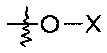
- Z für Wasserstoff steht.

4. Verbindung gemäß einem der Ansprüche 1 bis 3, die aus der Liste ausgewählt ist, die aus den folgenden Verbindungen besteht:
Beispiel 1: (2-Acetoxy-5-octanoyl-benzoyl)benzoylperoxid
Beispiel 2: (2-Ethoxycarbonyloxy-5-octanoyl-benzoyl)benzoylperoxid
Beispiel 3: (2-Propionyloxy-5-octanoyl-benzoyl)benzoylperoxid
Beispiel 4: (2-Butyryloxy-5-octanoyl-benzoyl)benzoylperoxid
Beispiel 5: (2-Isobutyryloxy-5-octanoyl-benzoyl)benzoylperoxid
Beispiel 6: [2-(2,2-Dimethyl-propionyloxy)-5-octanoyl-benzoyl]benzoylperoxid
Beispiel 7: (2-Pentanoyloxy-5-octanoyl-benzoyl)benzoylperoxid
Beispiel 8: [2-(2-Methyl-butyryloxy)-5-octanoyl-benzoyl]benzoylperoxid
Beispiel 9: [2-(3-Methyl-butyryloxy)-5-octanoyl-benzoyl]benzoylperoxid
Beispiel 10: (2-Hexanoyloxy-5-octanoyl-benzoyl)benzoylperoxid
Beispiel 11: [2-(2-Ethyl-butyryloxy)-5-octanoyl-benzoyl]benzoylperoxid
Beispiel 12: [2-(3,3-Dimethyl-butyryloxy)-5-octanoyl-benzoyl]benzoylperoxid
Beispiel 13: (2-Heptanoyloxy-5-octanoyl-benzoyl)benzoylperoxid
Beispiel 14: (2-Octanoyloxy-5-octanoyl-benzoyl)benzoylperoxid
Beispiel 15: (2-Nonanoyloxy-5-octanoyl-benzoyl)benzoylperoxid
Beispiel 16: (2-Cyclopropancarbonyloxy-5-octanoyl-benzoyl)benzoylperoxid
Beispiel 17: (2-Cyclobutancarbonyloxy-5-octanoyl-benzoyl)benzoylperoxid
Beispiel 18: (2-Cyclopentancarbonyloxy-5-octanoyl-benzoyl)benzoylperoxid
Beispiel 19: (2-Cyclohexancarbonyloxy-5-octanoyl-benzoyl)benzoylperoxid
Beispiel 20: (2-Benzoyloxy-5-octanoyl-benzoyl)benzoylperoxid
Beispiel 21: [2-(Adamantan-1-carbonyloxy)-5-octanoyl-benzoyl]benzoylperoxid
Beispiel 22: [2-(2-Adamantan-1-yl-acetoxy)-5-octanoyl-benzoyl]benzoylperoxid
Beispiel 23: (2-Methoxycabonyloxy-5-octanoyl-benzoyl)benzoylperoxid
Beispiel 24: (2-Propoxycarbonyloxy-5-octanoyl-benzoyl)benzoylperoxid
Beispiel 25: (2-Isopropoxycarbonyloxy-5-octanoyl-benzoyl)benzoylperoxid
Beispiel 26: (2-tert.-Butoxycarbonyloxy-5-octanoyl-benzoyl)benzoylperoxid
Beispiel 27: (2-Butoxycarbonyloxy-5-octanoyl-benzoyl)benzoylperoxid
Beispiel 28: (2-sec.-Butoxycarbonyloxy-5-octanoyl-benzoyl)benzoylperoxid
Beispiel 29: (2-Isobutoxycarbonyloxy-5-octanoyl-benzoyl)benzoylperoxid
Beispiel 30: (2-Pentoxycarbonyloxy-5-octanoyl-benzoyl)benzoylperoxid
Beispiel 31: [2-(1-Ethyl-propoxycarbonyloxy)-5-octanoyl-benzoyl]benzoylperoxid
Beispiel 32: [2-(2,2-Dimethyl-propoxycarbonyloxy)-5-octanoyl-benzoyl]benzoylperoxid
Beispiel 33: (2-Hexyloxycarbonyloxy-5-octanoyl-benzoyl)benzoylperoxid
Beispiel 34: (2-Heptyloxycarbonyloxy-5-octanoyl-benzoyl)benzoylperoxid
Beispiel 35: (2-Octyloxycarbonyloxy-5-octanoyl-benzoyl)benzoylperoxid
Beispiel 36: (2-Cyclopropoxycarbonyloxy-5-octanoyl-benzoyl)benzoylperoxid
Beispiel 37: (2-Cyclobutoxycarbonyloxy-5-octanoyl-benzoyl)benzoylperoxid
Beispiel 38: (2-Cyclopentoxycarbonyloxy-5-octanoyl-benzoyl)benzoylperoxid
Beispiel 39: (2-Cyclohexyloxycarbonyloxy-5-octanoyl-benzoyl)benzoylperoxid
Beispiel 40: (2-Phenoxycarbonyloxy-5-octanoyl-benzoyl)benzoylperoxid
Beispiel 41: (2-Acetoxymethoxy-5-octanoyl-benzoyl)benzoylperoxid
Beispiel 42: [2-(1-Acetoxy-ethoxy)-5-octanoyl-benzoyl]benzoylperoxid
Beispiel 43: bis(2-Acetoxy-5-octanoyl)-benzoylperoxid
Beispiel 44: bis(2-Propionyloxy-5-octanoyl)-benzoylperoxid
Beispiel 45: bis(2-Butyryloxy-5-octanoyl)-benzoylperoxid
Beispiel 46: bis(2-Isobutyryloxy-5-octanoyl)-benzoylperoxid
Beispiel 47: bis[2-(2,2-Dimethyl-propionyloxy)-5-octanoyl]-benzoylperoxid
Beispiel 48: bis(2-Pentanoyloxy-5-octanoyl-)benzoylperoxid
Beispiel 49: bis[2-(2-Methyl-butyryloxy)-5-octanoyl]-benzoylperoxid
Beispiel 50: bis[2-(3-Methyl-butyryloxy)-5-octanoyl]-benzoylperoxid
Beispiel 51: bis(2-Hexanoyloxy-5-octanoyl)-benzoylperoxid
Beispiel 52: bis[2-(2-Ethyl-butyryloxy)-5-octanoyl]-benzoylperoxid
Beispiel 53: bis[2-(3,3-Dimethyl-butyryloxy)-5-octanoyl]-benzoylperoxid
Beispiel 54: bis(2-Heptanoyloxy-5-octanoyl)-benzoylperoxid
Beispiel 55: bis(2-Octanoyloxy-5-octanoyl)-benzoylperoxid
Beispiel 56: bis(2-Nonanoyloxy-5-octanoyl)-benzoylperoxid
Beispiel 57: bis(2-Cylopropancarbonyloxy-5-octanoyl)-benzoylperoxid
Beispiel 58: bis(2-Cyclobutancarbonyloxy-5-octanoyl)-benzoylperoxid
Beispiel 59: bis(2-Cyclopentancarbonyloxy-5-octanoyl)-benzoylperoxid
Beispiel 60: bis(2-Cyclohexancarbonyloxy-5-octanoyl)-benzoylperoxid
Beispiel 61: bis(2-Benzoyloxy-5-octanoyl)-benzoylperoxid
Beispiel 62: bis[2-(Adamantan-l-carbonyloxy)-5-octanoyl]-benzoylperoxid
Beispiel 63: bis[2-(2-Adamantan-1-yl-acetoxy)-5-octanoyl]-benzoylperoxid
Beispiel 64: bis(2-Methoxycarbonyloxy-5-octanoyl)-benzoylperoxid
Beispiel 65: bis(2-Ethoxycarbonyloxy-5-octanoyl)-benzoylperoxid
Beispiel 66: bis(2-Propoxycarbonyloxy-5-octanoyl)-benzoylperoxid
Beispiel 67: bis(2-Isopropoxycarbonyloxy-5-octanoyl)-benzoylperoxid
Beispiel 68: bis(2-tert.-Butoxycarbonyloxy-5-octanoyl)-benzoylperoxid
Beispiel 69: bis(2-Butoxycarbonyloxy-5-octanoyl)-benzoylperoxid
Beispiel 70: bis(2-sec.-Butoxycarbonyloxy-5-octanoyl)-benzoylperoxid
Beispiel 71: bis(2-Isobutoxycarbonyloxy-5-octanoyl)-benzoylperoxid
Beispiel 72: bis(2-Pentoxycarbonyloxy-5-octanoyl)-benzoylperoxid
Beispiel 73: bis[2-(1-Ethyl-propoxycarbonyloxy)-5-octanoyl]-benzoylperoxid
Beispiel 74: bis[2-(2,2-Dimethyl-propoxycarbonyloxy)-5-octanoyl]-benzoylperoxid
Beispiel 75: bis(2-Hexyloxycarbonyloxy-5-octanoyl)-benzoylperoxid
Beispiel 76: bis(2-Heptyloxycarbonyloxy-5-octanoyl)-benzoylperoxid
Beispiel 77: bis(2-Octyloxycarbonyloxy-5-octanoyl)-benzoylperoxid
Beispiel 78: bis(2-Cyclopropoxycarbonyloxy-5-octanoyl)-benzoylperoxid
Beispiel 79: bis(2-Cyclobutoxycarbonyloxy-5-octanoyl)-benzoylperoxid
Beispiel 80: bis(2-Cyclopentoxycarbonyloxy-5-octanoyl)-benzoylperoxid
Beispiel 81: bis(2-Cyclohexyloxycarbonyloxy-5-octanoyl)-benzoylperoxid
Beispiel 82: bis(2-Phenoxycarbonyloxy-5-octanoyl)-benzoylperoxid
Beispiel 83: bis(2-Acetoxymethoxy-5-octanoyl)-benzoylperoxid
Beispiel 84: bis[2-(1-Acetoxy-ethoxy)-5-octanoyl]-benzoylperoxid

5. Verbindung gemäß einem der Ansprüche 1 bis 4 als Medikament.

6. Verbindung gemäß einem der Ansprüche 1 bis 5 zur Behandlung von Krankheiten oder Störungen, die mit dem Vorhandensein von Propionibacterium acnes verbunden sind.

7. Verbindung gemäß einem der Ansprüche 1 bis 4 zum Beimischen zu einer kosmetischen Zusammensetzung, **dadurch gekennzeichnet, dass** sie die Proliferation von pathogenen Keimen, insbesondere **P. acnes,** hemmt, die mit dem Auftreten von aknebedingten Störungen der Haut in Zusammenhang stehen.

## Claims

1. Compounds of general formula (I) below: 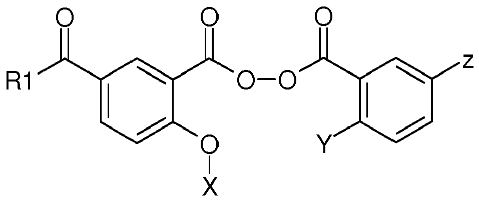 in which:
- R1 represents a lower alkyl or a higher alkyl;
- X represents a hydrogen or one of the following sequences: 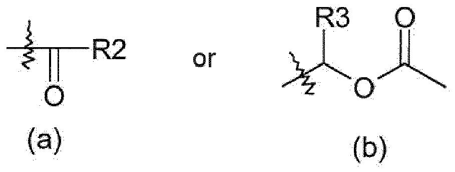
If X represents (a), R2 represents a lower alkyl, a higher alkyl, a cycloalkyl, a cycloalkylalkyl, a lower alkoxy, a higher alkoxy, a cycloalkyloxy, a cycloalkylalkoxy, an aryl or an aryloxy;
If X represents (b), R3 represents a hydrogen or a lower alkyl;
- Y represents a hydrogen or the following sequence: 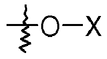
- Z represents a hydrogen or the following sequence:

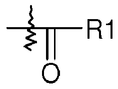

2. Compounds according to Claim 1, **characterized in that**:
- R1 represents a linear heptyl chain;
- X represents a hydrogen or one of the following sequences: 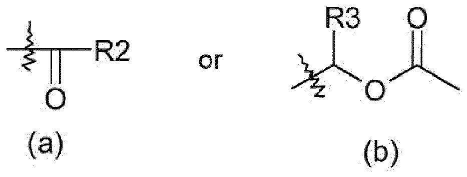 If X represents (a), R2 represents a lower alkyl, a higher alkyl, a cycloalkyl, a cycloalkylalkyl, a lower alkoxy, a higher alkoxy, a cycloalkyloxy, a cycloalkylalkoxy, an aryl or an aryloxy;
If X represents (b), R3 represents a hydrogen or a methyl group;
- Y represents a hydrogen or the following sequence: 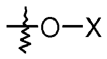
- Z represents a hydrogen or the following sequence:

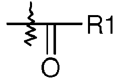

3. Compounds according to Claim 1 or 2, **characterized in that**:
- R1 represents a linear heptyl chain;
- X represents a hydrogen or one of the following sequences: 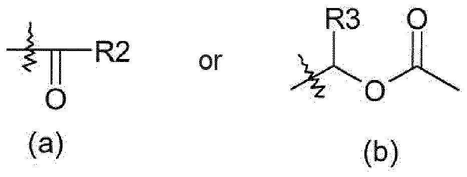 If X represents (a), R2 represents a lower alkyl, a higher alkyl, a cycloalkyl, a lower alkoxy or a higher alkoxy;
If X represents (b), R3 represents a hydrogen or a methyl group;
- Y represents a hydrogen or the following sequence: 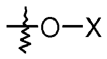
- Z represents a hydrogen.

4. Compound according to one of Claims 1 to 3, chosen from the list consisting of the following compounds:
Example 1: (2-acetoxy-5-octanoylbenzoyl)benzoyl peroxide
Example 2: (2-ethoxycarbonyloxy-5-octanoylbenzoyl)benzoyl peroxide
Example 3: (2-propionyloxy-5-octanoylbenzoyl)benzoyl peroxide
Example 4: (2-butyryloxy-5-octanoylbenzoyl)benzoyl peroxide
Example 5: (2-isobutyryloxy-5-octanoylbenzoyl)benzoyl peroxide
Example 6: [2-(2,2-dimethylpropionyloxy)-5-octanoylbenzoyl]benzoyl peroxide
Example 7: (2-pentanoyloxy-5-octanoylbenzoyl)benzoyl peroxide
Example 8: [2-(2-methylbutyryloxy)-5-octanoylbenzoyl]benzoyl peroxide
Example 9: [2-(3-methylbutyryloxy)-5-octanoylbenzoyl]benzoyl peroxide
Example 10: (2-hexanoyloxy-5-octanoylbenzoyl)benzoyl peroxide
Example 11: [2-(2-ethylbutyryloxy)-5-octanoylbenzoyl]benzoyl peroxide
Example 12: [2-(3,3-dimethylbutyryloxy)-5-octanoylbenzoyl]benzoyl peroxide
Example 13: (2-heptanoyloxy-5-octanoylbenzoyl)benzoyl peroxide
Example 14: (2-octanoyloxy-5-octanoylbenzoyl)benzoyl peroxide
Example 15: (2-nonanoyloxy-5-octanoylbenzoyl)benzoyl peroxide
Example 16: (2-cyclopropanecarbonyloxy-5-octanoylbenzoyl)benzoyl peroxide
Example 17: (2-cyclobutanecarbonyloxy-5-octanoylbenzoyl)benzoyl peroxide
Example 18: (2-cyclopentanecarbonyloxy-5-octanoylbenzoyl)benzoyl peroxide
Example 19: (2-cyclohexanecarbonyloxy-5-octanoylbenzoyl)benzoyl peroxide
Example 20: (2-benzoyloxy-5-octanoylbenzoyl)benzoyl peroxide
Example 21: [2-(adamantane-1-carbonyloxy)-5-octanoylbenzoyl]benzoyl peroxide
Example 22: [2-(2-adamantan-1-ylacetoxy)-5-octanoylbenzoyl]benzoyl peroxide
Example 23: (2-methoxycarbonyloxy-5-octanoylbenzoyl)benzoyl peroxide
Example 24: (2-propoxycarbonyloxy-5-octanoylbenzoyl)benzoyl peroxide
Example 25: (2-isopropoxycarbonyloxy-5-octanoylbenzoyl)benzoyl peroxide
Example 26: (2-tert-butoxycarbonyloxy-5-octanoylbenzoyl)benzoyl peroxide
Example 27: (2-butoxycarbonyloxy-5-octanoylbenzoyl)benzoyl peroxide
Example 28: (2-sec-butoxycarbonyloxy-5-octanoylbenzoyl)benzoyl peroxide
Example 29: (2-isobutoxycarbonyloxy-5-octanoylbenzoyl)benzoyl peroxide
Example 30: (2-pentoxycarbonyloxy-5-octanoylbenzoyl)benzoyl peroxide
Example 31: [2-(1-ethylpropoxycarbonyloxy)-5-octanoylbenzoyl]benzoyl peroxide
Example 32: [2-(2,2-dimethylpropoxycarbonyloxy)-5-octanoylbenzoyl]benzoyl peroxide
Example 33: (2-hexyloxycarbonyloxy-5-octanoylbenzoyl)benzoyl peroxide
Example 34: (2-heptyloxycarbonyloxy-5-octanoylbenzoyl)benzoyl peroxide
Example 35: (2-octyloxycarbonyloxy-5-octanoylbenzoyl)benzoyl peroxide
Example 36: (2-cyclopropoxycarbonyloxy-5-octanoylbenzoyl)benzoyl peroxide
Example 37: (2-cyclobutoxycarbonyloxy-5-octanoylbenzoyl)benzoyl peroxide
Example 38: (2-cyclopentoxycarbonyloxy-5-octanoylbenzoyl)benzoyl peroxide
Example 39: (2-cyclohexyloxycarbonyloxy-5-octanoylbenzoyl)benzoyl peroxide
Example 40: (2-phenoxycarbonyloxy-5-octanoylbenzoyl)benzoyl peroxide
Example 41: (2-acetoxymethoxy-5-octanoylbenzoyl)benzoyl peroxide
Example 42: [2-(1-acetoxyethoxy)-5-octanoylbenzoyl]benzoyl peroxide
Example 43: bis(2-acetoxy-5-octanoyl)benzoyl peroxide Example 44: bis(2-propionyloxy-5-octanoyl)benzoyl peroxide
Example 45: bis(2-butyryloxy-5-octanoyl)benzoyl peroxide
Example 46: bis(2-isobutyryloxy-5-octanoyl)benzoyl peroxide
Example 47: bis[2-(2,2-dimethylpropionyloxy)-5-octanoyl]benzoyl peroxide
Example 48: bis(2-pentanoyloxy-5-octanoyl)benzoyl peroxide
Example 49: bis[2-(2-methylbutyryloxy)-5-octanoyl]benzoyl peroxide
Example 50: bis[2-(3-methylbutyryloxy)-5-octanoyl]benzoyl peroxide
Example 51: bis(2-hexanoyloxy-5-octanoyl)benzoyl peroxide
Example 52: bis[2-(2-ethylbutyryloxy)-5-octanoyl]benzoyl peroxide
Example 53: bis[2-(3,3-dimethylbutyryloxy)-5-octanoyl]benzoyl peroxide
Example 54: bis(2-heptanoyloxy-5-octanoyl)benzoyl peroxide
Example 55: bis(2-octanoyloxy-5-octanoyl)benzoyl peroxide
Example 56: bis(2-nonanoyloxy-5-octanoyl)benzoyl peroxide
Example 57: bis(2-cyclopropanecarbonyloxy-5-octanoyl)benzoyl peroxide
Example 58: bis(2-cyclobutanecarbonyloxy-5-octanoyl)benzoyl peroxide
Example 59: bis(2-cyclopentanecarbonyloxy-5-octanoyl)benzoyl peroxide
Example 60: bis(2-cyclohexanecarbonyloxy-5-octanoyl)benzoyl peroxide
Example 61: bis(2-benzoyloxy-5-octanoyl)benzoyl peroxide
Example 62: bis[2-(adamantane-1-carbonyloxy)-5-octanoyl]benzoyl peroxide
Example 63: bis[2-(2-adamantan-1-ylacetoxy)-5-octanoyl]benzoyl peroxide
Example 64: bis(2-methoxycarbonyloxy-5-octanoyl)benzoyl peroxide
Example 65: bis(2-ethoxycarbonyloxy-5-octanoyl)benzoyl peroxide
Example 66: bis(2-propoxycarbonyloxy-5-octanoyl)benzoyl peroxide
Example 67: bis(2-isopropoxycarbonyloxy-5-octanoyl)benzoyl peroxide
Example 68: bis(2-tert-butoxycarbonyloxy-5-octanoyl)benzoyl peroxide
Example 69: bis(2-butoxycarbonyloxy-5-octanoyl)benzoyl peroxide
Example 70: bis(2-sec-butoxycarbonyloxy-5-octanoyl)benzoyl peroxide
Example 71: bis(2-isobutoxycarbonyloxy-5-octanoyl)benzoyl peroxide
Example 72: bis(2-pentoxycarbonyloxy-5-octanoyl)benzoyl peroxide
Example 73: bis[2-(1-ethylpropoxycarbonyloxy)-5-octanoyl]benzoyl peroxide
Example 74: bis[2-(2,2-dimethylpropoxycarbonyloxy)-5-octanoyl]benzoyl peroxide
Example 75: bis(2-hexyloxycarbonyloxy-5-octanoyl)benzoyl peroxide
Example 76: bis(2-heptyloxycarbonyloxy-5-octanoyl)benzoyl peroxide
Example 77: bis(2-octyloxycarbonyloxy-5-octanoyl)benzoyl peroxide
Example 78: bis(2-cyclopropoxycarbonyloxy-5-octanoyl)benzoyl peroxide
Example 79: bis(2-cyclobutoxycarbonyloxy-5-octanoyl)benzoyl peroxide
Example 80: bis(2-cyclopentoxycarbonyloxy-5-octanoyl)benzoyl peroxide
Example 81: bis(2-cyclohexyloxycarbonyloxy-5-octanoyl)benzoyl peroxide
Example 82: bis(2-phenoxycarbonyloxy-5-octanoyl)benzoyl peroxide
Example 83: bis(2-acetoxymethoxy-5-octanoyl)benzoyl peroxide
Example 84: bis[2-(1-acetoxyethoxy)-5-octanoyl]benzoyl peroxide

5. Compound according to one of Claims 1 to 4, as a medicament.

6. Compound according to one of Claims 1 to 5, for the treatment of pathologies or disorders linked to the presence of *Propionibacterium acnes.*

7. Compound according to one of Claims 1 to 4, for incorporation into a cosmetic composition, **characterized in that** it inhibits the proliferation of pathogenic microorganisms involved in the appearance of acne-type skin disorders, especially ***P. acnes.***
